# EUROPEAN PATENT SPECIFICATION

(11) **EP 0 940 133 B1**
(45) Date of publication and mention of the grant of the patent: **11.06.2003**
(21) Application number: 99104448.8
(22) Date of filing: 05.03.1999
(51) Int. Cl.: A61F 13/15

(54) **Sanitary napkin having flaps with zones of differential stiffness**
Damenbinde mit Flügeln mit Zonen unterschiedlicher Steifheit
Serviette hygiénique pourvue de rabats avec des zones de rigidités diffèrentes

(30) Priority: 05.03.1998 US 35599
(43) Date of publication of application: 08.09.1999
(73) Proprietor: McNEIL-PPC, Inc., Skillman, New Jersey 08558 (US)
(72) Inventor: Kessler, Tyra, Ephrata, PA 17522 (US); Glasgow, Tara, Yardley, PA 19067 (US); Mavinkurve, Pramod S., Princeton, NJ 08540 (US)
(74) Representative: Groening, Hans Wilhelm, Dipl.-Ing.

(56) References cited:
- EP-A- 0 571 981
- WO-A-95/20932
- WO-A-97/00655
- GB-A- 2 276 552
- US-A- 5 154 715

## Description

### Field of the Invention

The present invention relates to an absorbent article for absorbing body fluids and more particularly to a sanitary napkin having laterally extending flaps which are affixed to and extend from the napkin. The flaps are provided with zones of differential stiffness, which when folded around a crotch region of a wearer's undergarment create a preferential bending zone in the flap which defines a longitudinally extending hinge region adjacent to a line of juncture between the flap and the main absorbent body of the napkin.

### Background of the Invention

Sanitary napkins having side flaps (also commonly referred to in the art as tabs, wings, or side panels) which are adapted to be folded about the edges of a crotch portion of a wearer's undergarment are well known in the art and such are disclosed in U.S. Pat. No 4,258,343 to McNair, U.S. Pat. No. 4,589,876 to Van Tilburg and U.S. Pat. No. 4,608,047 to Mattingly. The McNair patent discloses sanitary napkins having flaps which are relatively inflexible and which have a single flexible axis where the flaps are affixed to the side edges of the napkin. The Van Tilburg and Mattingly patents disclose sanitary napkins having flexible side flaps. The Van Tilburg further discloses that the flexible flaps have two axes of flexibility, which are intended to serve as bending or folding lines wherein each flap has a first longitudinally extending flexible axis at a line of juncture where the flap adjoins the body of the napkin and a second longitudinally extending flexible axis created in the flap outboard from the line of juncture. The stated purpose of providing the flaps with these two flexible axes is to enable the flap to fold upward from the first flexible axis towards the user's groins and to then enable the flap to fold downwardly over the edge of the user's undergarment at the second flexible axis. The second flexible axis is intended to form a gasket-like seal against the body of the user.

Flexible flaps have been found to create problems for the user in that while attempting to place the flaps into an undergarment, the flaps often fold onto themselves causing adjacent regions of the flaps having positioning adhesive to adhere to each other, thus rendering the product unusable. In addition, flexible flaps have been found to be difficult for the user to control when folding around the edges of the undergarment.

WO 95/20932 (Osborn et al.) discloses sanitary napkins which comprise a pair of side extensions one of which is located along each longitudinal side edge of the main body portion. In one embodiment said side extensions exhibit at least two regions with different directions of extensibility, the first region adjacent to the main body portion having a longitudinal extensibility, and a second region at least some portions of which lie laterally outboard of the longitudinal side edges, which is extensible primarily in the traverse direction.

In another embodiment Osborn et al. describe side extensions the distal edges of which comprise stiffer regions in case a topsheet is used which comprises an appertured web material with a strainible network exhibiting elastic-like behavior without edit elastic materials.

According to Osborn et al. side extensions which have at least three regions with different stiffnesses and flexibility characteristics are preferred. The most flexible region preferably consists of two zones of longitudinal extensibility which both extend along the longitudinal edges of the main body portion of the napkin. The distal edges define stiffer regions of the side extensions. An intermediate region of the side extension is disposed between the above described regions. This intermediate region is more flexible than the distal edge region, but less flexible than the region adjacent to the main body portion.

WO 97/00655 issued to Redwine et al. discloses an absorbent article having side wrapping elements which are either integral extensions of the components of the main body portion, or separate components that are joint to the garment-facing side of the main body portion. The side wrapping elements according to Redwine et al. are provided with at least one zone of extensibility, preferably with two spaced apart zones of extensibility. Also, Redwine et al. describe a sanitary napkin which has at least a three distinct regions with different bending properties. The first region comprises a portion of the main body portion that contains the absorbent core and which has the highest bending modulus. The second region comprises an intermediate region of the side wrapping element, and has a bending modulus which is between the bending moduli of the first and the third region. This third region comprises the bending zone and represents the most flexible region.

WO 99/29274 (Marshall at al.) also describes a sanitary napkin having flaps which extend laterally outward from the longitudinal sides of the base member. These flaps comprise at least one zone of extensibility wherein at least a portion of the zone of extensibility is spaced longitudinally away from the flap transverse outer line.

Finally, EP 1 070 493 A2 (Lavash et al.) describes an absorbent article for wearing in an undergarment which also comprises a pair of flaps. In order to eliminate or reduce the stresses concentrated along the fold where the flap changes from being disposed on the body side of the panty to being located on the underside of the panty the sanitary napkin of Lavash at al. is provided with zones of differential extensibility. These zones are preferably located at those points where the edges of the flaps intersect the edges of the panty when the sanitary napkin is warm.

### Summary of the Invention

It is an object of this invention to provide a novel sanitary napkin having side flaps with a greater ease of attachment than conventional sanitary napkins.

It is another object of this invention to provide a sanitary napkin having side flaps which has an enhanced ability to remain attached to a wearer's undergarment during use.

It is another object of this invention to provide a sanitary napkin having side flaps which in use has enhanced resilience and resistance to deformation due to the laterally compressive forces of a wearer's thighs.
This object is achieved by an absorbent article of claim 1.

In accordance with the present invention, there has been provided a novel sanitary napkin comprising a fluid pervious topsheet, a fluid impervious backsheet, an absorbent core between the topsbeet and the backsheet, the topsheet and the backsheet being sealed together around their respective peripheral edges to enclose the absorbent core, the napkin having first and second longitudinal side edges and first and second transverse edges, the napkin further comprising side flaps which extend laterally from the longitudinal side edges along a line of juncture of the flop and the longitudinal side edge of the sanitary napkin, each side flap having a flexible portion which is adjacent the line of juncture of the flap and a distal region which extends laterally outward from and is continuous with the proximal region. The distal region comprises a stiffened portion which comprises a plurality of individual stiffened portions which are separated by one or more laterally extending flexible axes, and which has an increased resistance to lateral compressive forces relative to the flexible portion of the flap. Preferred embodiments of the invention are disclosed in claims 2 to 9.

### Brief Description of the Drawings.

Figure 1 is a top plan view of a sanitary napkin which is not according to the present invention.
Figure 2. is a cross sectional view taken along line 2-2 in Figure 1.
Figure 3 is a top plan view of an embodiment of the sanitary napkin of the present invention.
Figure 4 is a top plan view of another embodiment of the sanitary napkin of the present invention.
Figure 5 is a fragmentary coronal view showing the sectioned sanitary napkin of Figures 1, 3 or 4 in place on a user.
Figure 6 is a top plan view of a flap of another embodiment of the santary napkin of the present invention having a stiffened portion in the form of an embossing pattern.
Figure 7 is a top plan view of a flap of another embodiment of the santary napkin of the present invention having a stiffened portion in the form of another embossing pattern.
Figure 8 is a top plan view of a flap of another embodiment of the sanitary napkin of the present invention having a stiffened portion in the form of another embossing pattern.
Figure 9 is a top plan view of a flap of another embodiment of the sanitary napkin of the present invention having a stiffened portion in the form of another embossing pattern.
Figure 10 is a bottom plan view of a sanitary napkin which is not regarding to the present invention having flaps affixed to the barrier layer inward from the longitudinal side edges of the napkin.
Figure 11 is a cross sectional view taken along line 11-11 in Figure 10.
Figure 12 is a fragmentary coronal view showing the sectioned sanitary napkin of Figures 10 in place on a user.
Figure 13 is a top plan view of another embodiment of the sanitary napkin of the present invention.

### Detailed Description

The present invention is directed to a novel sanitary napkin having a pair of oppositely attached laterally extending flaps along or adjacent to each longitudinal side edge along a line of juncture, each of the flaps having zones of differential stiffness which create lateral stiffness in a distal region of each flap and a flexible portion in a proximal region of the flap to provide a preferential bending zone or hinge region in the proximal region adjacent the line of juncture of the flap. Flaps having zones of differential stiffness provide enhanced control when a user folds the flaps around a crotch portion of an undergarment. More specifically, when a flap is wrapped around the panty elastic, it does not bend at a single hinge line but actually bends across a certain distance that is determined by the thickness of the edge portion of the panty (or the panty elastic) and the contour of the crotch. Due to the contour shape, the flap bends primarily in the longitudinally oriented direction, but also has some bending components in the transverse direction. As used herein, "bending in the longitudinal direction" refers to bending that occurs primarily in the longitudinal direction. In accordance with the present invention, the stiffened distal region of the flap provides greater control when folding the flaps over the edges of the undergarment and the flexible portion of the flap enables the flap, when folded over the panty crotch, to form a smooth curve over the edges of the panty. In a preferred embodiment of the present invention, the zones of differential stiffness create lateral stiffness in the flap but do not increase the longitudinal stiffness of the flap.

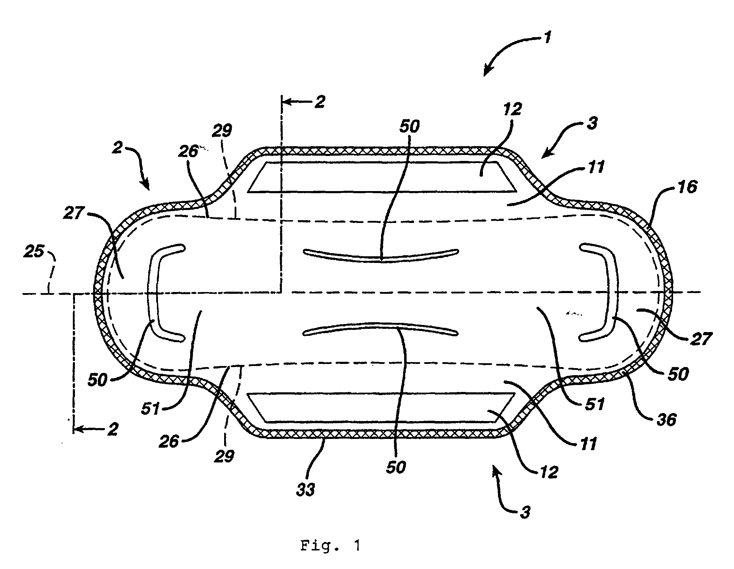

In accordance with the present invention there has been provided a novel sanitary napkin having flaps which are affixed or otherwise attached to the longitudinal side edges of the central absorbent element of the napkin, the flaps having zones of differential stiffness. Referring to Figure 1, a sanitary napkin 1 comprises an elongated main body 2 having a longitudinal centerline 25, opposite longitudinal side edges 26 and opposite transverse end regions 27 and two flaps 3, each flap extending laterally outward from a central region of each longitudinal side edge 26.

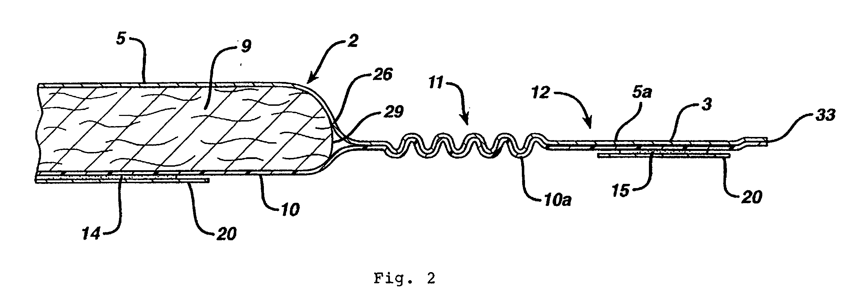

Referring to Figure 2, the main body 2 is made in a layered manner and generally includes, from the top down, an upper, body facing, flexible, fluid permeable cover layer 5, an optional transfer layer (not shown), an absorbent layer 9, and a lower, garment facing flexible liquid barrier layer 10. The flaps 3 have a barrier layer 10a which is preferably continuous with the barrier layer 10 of the body 2 and a cover layer 5a which is preferably continuous with the cover layer 5 of the body 2. The barrier layer 10 is provided with a longitudinally extending adhesive band 14 for adhesion of the main body 2 to a user's undergarment. The barrier layer 10a is also provided with adhesive patches 15 on the flaps 3 for securing the flaps 3 to underside of the user's undergarment and thereby provide additional security for the placement of the napkin 1. As is conventional, silicon coated paper 20 may be provided as a temporary protective cover for the adhesive strips 14 and patches 15.

The cover layer 5 may be any flexible, liquid permeable, layer of material including woven or nonwoven fabric or perforated polymeric film.

An optional transfer layer (not shown), may be under the cover layer 5, and is preferably a high void volume porous structure capable of fast liquid acquisition. The transfer layer may be made of cellulose fibers, rayon fibers, polypropylene fibers, polyester fibers, polyethylene fibers, bi-component fibers and the like and combinations thereof. The ability of the transfer layer to rapidly absorb liquid from the cover layer 5 prevents the body exudate from pooling on the cover layer 5 and reduces the likelihood of body exudate leaking past the side edges of the sanitary napkin 1. The transfer layer is preferably glued, thermobonded or otherwise adhered to the cover layer 5 above and to the absorbent layer 9 below.

The absorbent layer 9 may be made of cellulosic pulp fluff, sphagnum moss particles, superabsorbent polymers, synthetic fibers and the like and combinations thereof. This layer provides for the actual absorption and retention of the collected fluid. Thus, while the transfer layer provides a rapid absorption of liquid from the surface of the napkin, the absorbent layer 9, more slowly acquires and retains the liquid from the transfer layer.

The barrier layer 10 may be made of any flexible liquid impermeable material, and is preferably polyethylene. This layer is below the absorbent layer 9 and is impermeable to liquid. Thus, liquid collected by the absorbent layer 9 cannot egress from the absorbent structure to the user's undergarment (not shown).

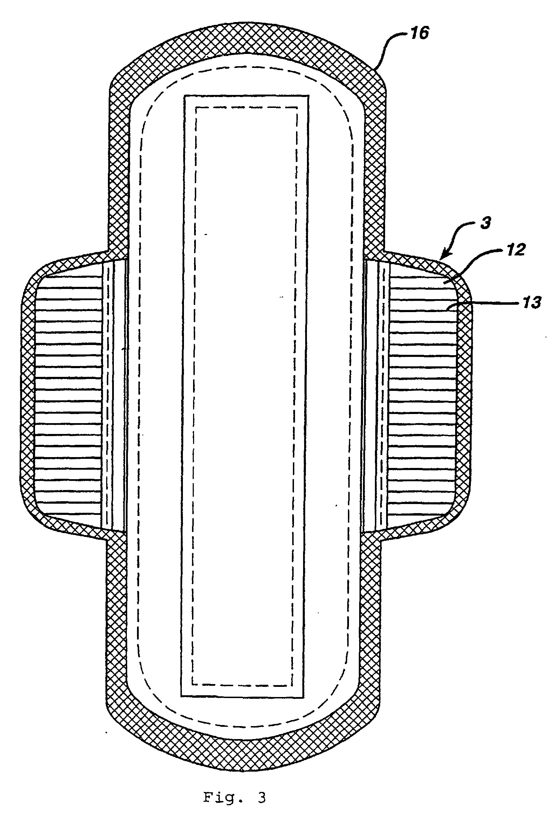

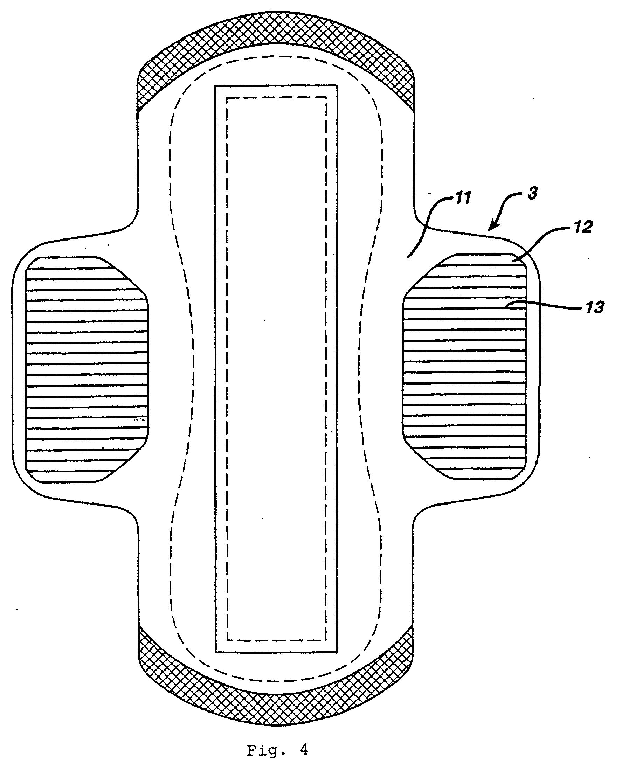

Referring to Figures 3 and 4, in manufacturing the napkin 1, the barrier layer 10 and the cover layer 5 are generally cut to substantially the same size, having elongate main body areas which are slightly wider and longer than the transfer layer (not shown) and the absorbent layer 9, and with two laterally extending flaps 3 as discussed above. The napkin 1 is sealed around the edges, preferably with a continuous thermal or adhesive bond 16 between the barrier layer 10 and the cover layer 5 to form a flange 36. This bonding is preferably also performed along the outer perimeter of the flaps 3, along the outer peripheral edge of the main body 2, and optionally between the main body 2 and the flaps 3 (not shown), to form a continuous closure line around the transfer layer (not shown) and the absorbent layer 9.

The flaps 3 have a length dimension which is defined as extending laterally outward from the main body 2 portion of the sanitary napkin 1 in a direction substantially perpendicular to a longitudinal centerline 25 of the napkin and is measured from the line of juncture 29 where the flap is affixed to the napkin and extends to the distal end 33 of the freely extending flap. The flaps 3 also have a width dimension which extends in the plane of the flap in a direction substantially parallel to the longitudinal centerline 25 of the napkin and generally orthogonal to the length dimension of the flap. The flaps 3 have zones of differential stiffness which comprise a flexible portion 11 adjacent to and extending laterally outward from the line of juncture 29 and a stiffened portion 12 adjacent to and extending laterally outward from the flexible portion 11 to the distal end 33 of the flap 3.

The flexible portion 11 of flap 3 has a length which extends along the entire line of juncture 29 of the flap 3 and the main body 2 in the proximal region of the flap 3. The flexible portion 11 has a width which extends from the line of juncture 29 to the stiffened portion 12. The width of the flexible portion 11 is sufficient to permit the flexible portion 11 to form a smooth curve when folded around the edge of the user's undergarment. The width of the flexible portion 11 is preferably greater than about 1 millimeter and is generally in a range of about 1 to 25 millimeters, preferably between about 2 millimeters and 10 millimeters.

The stiffened portion 12 of flap 3 covers substantially the entire body of the flap 3 excluding of course the flexible portion 11. The stiffened portion 12 need not extend fully to the opposite transverse edges or to the distal edge 33 of the flap and may be inset slightly from these edges provided of course that the flap maintains its enhanced lateral stiffness over a substantial portion of the flap. The stiffened portion 12 may terminate at the flexible portion 11 in a substantially straight line, as illustrated in Figure 3, substantially parallel to the line of juncture 29 or preferably may terminate in an arcuate line, as illustrated in Figure 4, which allows the flap 3 to more easily conforms to the arcuate shape of a crotch region of a wearer's undergarment. In a most preferred embodiment, the absorbent core has a substantially hourglass shape, i.e. wider transverse end regions 27 and narrower center regions with arcuate longitudinal sides and the regions of enhanced stiffness terminate in an arcuate line which is substantially parallel to the arcuate longitudinal sides of the absorbent core. The stiffened portion 12 preferably extends across the entire width of the flap. The stiffened portion 12 is discontinuous, provided of course that any discontinuous regions do not provide a second preferential bending zone across the length of the flap.

The stiffened portion 12 of the flap 3 has a higher resistance to laterally compressive forces relative to the flexible portion 11. The stiffened portion 12 should have a resistance to lateral compression which is generally between 1.5 and 200 times greater that a resistance to lateral compression in the flexible portion 11 of the flap 3 and is preferably between 1.5 and 50 times greater and most preferably between 2 and 8 times greater. The relative stiffness or flexibility of a flap's resistance to lateral compression can be characterized by measuring its resistance to bending and can be conveniently determined with a Genuine Gurley TM Bending Resistance/Stiffness Tester, Model 4171 D, which is commercially available from Gurley Precision Instruments, Inc., Troy New York. When samples a stiffened portion 12 of the present flaps 3 were evaluated on this apparatus, a 2.52 centimeter by 5.04 centimeter sample had a resistance tc bending in a range of from 0.75 grams to about 250 grams, preferably from 2 grams to 50 grams, and most preferably from 5 grams to 20 grams. Using the same procedure, the flexible portion 11 of the flap had a resistance to bending in a range of from 0.5 grams to 8 grams, and was preferably about 3 grams.

Referring to Figures 3 and 4, which display napkins regarding to the invention, the stiffened portion 12 comprises a plurality of individual stiffened portions 12 which are separated by one or more laterally extending flexible axes 13, such as, for example in a striped pattern wherein two or more stripes of stiffened portion 12 extend substantially across the length dimension of the flap. By separating the stiffened portions 12 across the width of the flap, the distal region of the flap resists compression due to the application of laterally compressive forces, yet is flexible along the width of the flap (i.e. it would not inhibit longitudinal deformation of the sanitary napkin

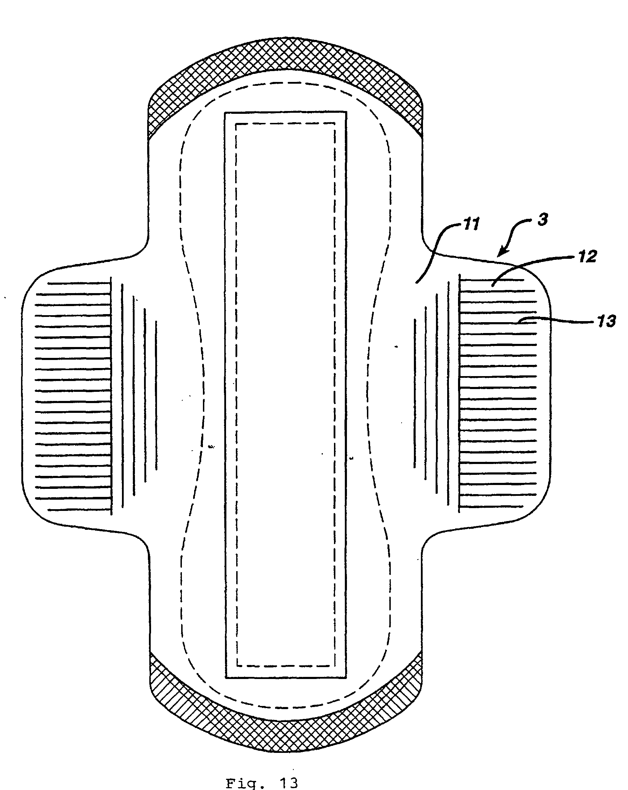

1). This embodiment enables the sanitary napkin 1 to easily conform to a user's body when placed in an undergarment but provides resistance to laterally compressive forces. Thus, in accordance with the invention, the stiffened portion 12 of the flap is freely flexible across its width but resists bending across its length. In this manner, each flap not only preferentially bends in the flexible portion 11 adjacent to the line of juncture 29 between the flap and the main absorbent body of the napkin and resists lateral bending in the stiffened portion 12 of the flap 3, but is also flexible in a longitudinal direction of the napkin. As illustrated in Figure 13, the flexible portion 11 of the flap 3 may optionally include one or more stripes of stiffening material oriented across the width of the flap 3. These stripes of stiffening material provide a resistance to bending across the width of the flap 3 but do not impart a resistance to bending across the length of the flap 3 in the flexible portion 11.

The stiffened portion 12 of the flap 3 may be created by the incorporating into the flap any material which increases the stiffness of the flap relative to regions of the flap which to not contain this material. Examples of suitable materials include, but are not limited to tissue, non-woven fabric, polymer film, airlaid pulp, polymeric foam, non-pressure sensitive adhesive, embossments and the like and combinations thereof. The added materials are preferably flexible so as not to create discomfort to the user of the napkin and will generally range in thickness from about 0.02 millimeters to about 2 millimeters, preferably from about 0.025 millimeters (about 1 mil) to about 0.25 millimeters (about 10 mils) thick. While it is preferred that the materials incorporated into the flap be non-wicking, it is possible to use material which are ordinarily subject to wet collapse since they are not in contact with body fluids. It is also preferred that the materials incorporated into the flaps 3 be resilient. By providing resilience to the distal regions of the flaps 3, when the flaps 3 are folded under the user's undergarment and affixed to the undergarment in a central region of the absorbent napkin, they provide the napkin with greater recovery to lateral compressive forces. That is, since it is the central region of a sanitary absorbent napkin which is subjected to the laterally compressive forces of a user's thighs, the resilient material in the flaps 3, when folded and adhered under the napkin, enables the sanitary napkin 1 to better maintain its original shape and reduce bunching, twisting and roping.

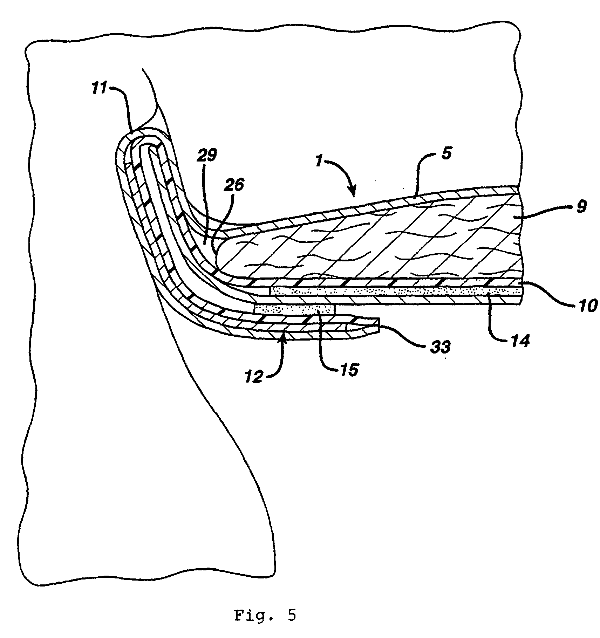

An important consideration when constructing a sanitary napkin 1 having flaps 3 with zones of differential stiffness is to determine where the flexible portion 11 and the stiffened portion 12 are to be located in the flap. By controlling where the preferential fold region or hinge zone occurs in the flap relative to the central absorbent element, the ability of the flap to gather or maintain the edges of the panty towards the edges of the central absorbent element can be more easily controlled. That is, the closer the two opposing flexible zones are together, the greater the tendency for the flaps 3 to gather the edges of the undergarment towards the longitudinal centerline 25 of the napkin. It is generally known in the art that an average undergarment has a medial crotch width of about 7.5 centimeters It is also known that the crotch region of an undergarment widens substantially towards the front and end regions. Thus, the distance between the preferential fold region created by flexible portions 11 in the oppositely attached flaps 3 is between about 7 centimeters and 8.5 centimeters It has been found that by providing the absorbent article with oppositely attached flaps 3 having zones of differential stiffness wherein the flexible portions 11 in each respective flap are separated one from another by a distance of about 7 to 8.5 centimeters, that the flaps 3 easily gather the wider portions of the undergarment inward towards the longitudinal side edges 26 of the napkin and yet enables the flaps 3 to be secured around the side edges of the undergarment and adhesively secured under the absorbent element of the napkin rather than under an adjacent portion of the flap as illustrated in Figure 5. The expedient of providing zones of differential stiffness in the flaps 3 has also been found to aid in the ease of attachment, reduce wrinkles and creases in the flap and consequently helps the flap stay in place in a wearer's undergarment.

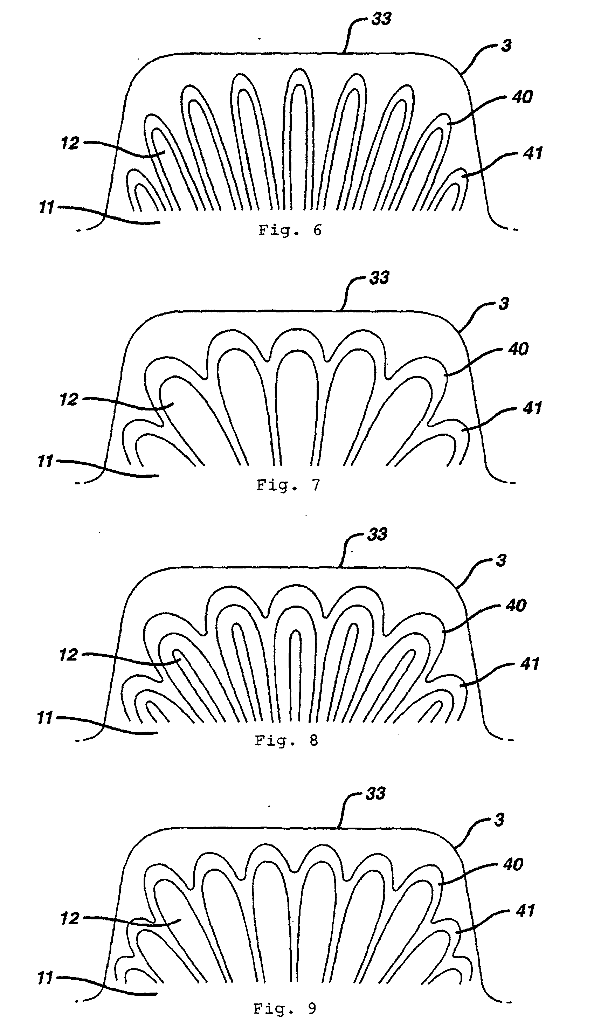

Referring to Figures 6-9, in another preferred embodiment of the present invention, the flaps 3 are provided with zones of differential stiffness which are created by embossing the distal end regions of the flaps 3 in a pattern wherein the embossments 40 start slightly outward from the line of juncture 29, preferably from 2 millimeters to 25 millimeters, most preferably from 3 millimeters to 10 millimeters outward from the line of juncture 29. The embossments 40 preferably terminate slightly inward from the distal edge of the flap, preferably from about 2 millimeters to about 10 millimeters inward from the distal edge of the flap. A preferred pattern of embossment is a series of loops 41 which start adjacent the flexible portion 11 and which extend outward toward the distal edge of the flap and curve back toward the flexible portion 11 where they terminate.

In another preferred embodiment of the present invention, the central absorbent core has a width which exceeds the width of a user's labia majora. While labial widths can vary widely from user to user, it has been found that an absorbent core width of at least 70 millimeters, preferably about 75 millimeters, generally exceeds the width of most women's labia majora. Since the medial crotch width of panties is generally about 7.5 centimeters. The flaps 3 of the present invention, when used on a sanitary napkin 1 having a core width of at least 70 millimeters and when used in a conventional panty, more easily folds around the edges of the panty crotch edge and have a greater ease of attachment than conventional sanitary napkins with flaps. As discussed above, since the flexible portion 11 of the flap determines where the flap will fold. Thus, the flaps 3 can be adapted to assure that they are affixed to the underside of the user's undergarment in a location which is under the absorbent element rather than under an adjacent portion of the flap. A core width of at least 70 millimeters sufficiently separates the flexible portions 11 of the flaps 3 to reduce or eliminate the stresses which are applied to the flaps 3 by the side edges of a user's undergarment (which often contain an elastic element) and thereby provides the flaps 3 with an enhanced ability to remain attached to a wearer's undergarment during use.

In another embodiment of the present invention, the sanitary absorbent articles have an absorbent element formed by an upper body facing cover layer 5, a lower, garment facing barrier layer 10 and an absorbent core between the cover layer 5 and the barrier layer 10, as previously defined, wherein the absorbent element further comprises at least one conforming means. More specifically, the absorbent element has a central region, a first or anterior end region and a second or posterior end region. The central region has longitudinal edges coincident with the longitudinal edges of the absorbent element and first and second distal ends opposite each other defining an area that is sufficient to cover at least the woman's vestibule and labia majora in use. The first or anterior end region, extends from the first distal end of the central region and is adapted to cover at least a portion of the woman's mons pubis in use. The second or posterior end region extends from the second distal end of the central region and is adapted to cover at least a portion of the woman's posterior perineum in use. The absorbent element has at least one conforming means located within at least one of the end regions of the absorbent element, i.e. either in the first end region or in the second end region or both the first and second end regions. Referring again to Figure 1, the conforming means comprises a stiffened element 50 and a non-stiffened region 51. The non-stiffened region 51 is adjacent the distal end of the central region and between the central region and the stiffened element 50. The stiffened element 50 is located inward of the transverse and longitudinal edges of the absorbent element and between the non-stiffened region 51 and the distal end of the absorbent element. The non-stiffened region 51 extends transversely across the end region, generally perpendicular to the central longitudinal axis of the absorbent product from one longitudinal edge of the absorbent element to the opposite longitudinal edge of the absorbent element. The stiffened element 50 extends transversely across the end region, generally perpendicular to the central longitudinal axis of the absorbent product, at least at its intersection with that axis, and centrally occupies at least 50% of the width of the absorbent element. The stiffened element 50, in use, resists transverse bunching, i.e. it has a higher resistance to laterally compressive forces relative to the non-stiffened region 51. In combination, the stiffened element 50 and the non-stiffened region 51, in use, enables the end region of the absorbent product to preferentially bend longitudinally thereby providing the napkin with the ability to closely adapt to and fit the contours of a user's body. The stiffened element 50 may also act as a barrier to fluid wicking and guide the fluid so that it is retained within the confines of the absorbent element. The conforming means thereby provides an axis of bending, that coincides with a transverse axis of the napkin, a resistance to bending and compression orthogonal to that axis, i.e., along the longitudinal axis of the napkin and a relatively unconstrained region that may puff and cuff outward to fit and conform to the body. The stiffened element 50 may optionally be adapted to prevent leakage of fluid from the edges, as hereinafter described. The stiffened element 50 may of itself be a bending means, or may include a separate bending means, such as an embossed channel, or may comprise a projection, pleat, slit, hinge means or thinned area, that provides an axis of flexibility, coincident with the axis of bending of such bending means as well as providing stiffening and compression resistance orthogonal to the axis of bending.

The center region may optionally contain one or more stiffening means, such as extra absorbent material, a sphagnum-moss containing insert, an embossed channel having a component parallel to the central longitudinal axis, and combinations thereof. Embossed channels are preferably located between the central longitudinal axis and the longitudinal edge, provided of course that the stiffening means in the central region is spaced apart from the stiffened element 50 in the end region. The stiffening means maintains the center of the absorbent element in a relatively flat profile along the longitudinal axis, and resists bending of the absorbent element transversely to that axis, so as to effectively conform to the body in that region, resist transverse bending and bunching and thereby prevent leakage of fluid from the absorbent element. In a most preferred embodiment, the center region comprises additional absorbent material relative to the end regions of the absorbent element, the additional absorbent material having a pair of arcuate channels embossed therein to form a stiffened center region and conforming means located within both end regions of the absorbent element, the conforming means comprising an embossed channel. It is preferred that the width dimension of the flap be equal to or exceed the length of the embossed channel. A preferred embossed channel length is about 75 millimeters.

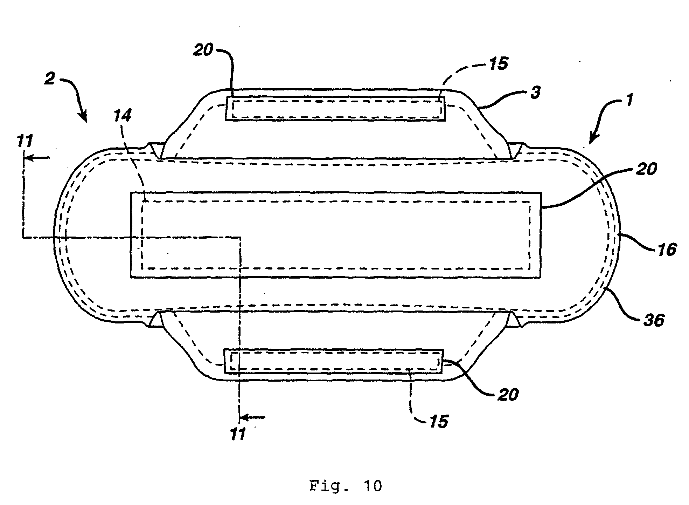

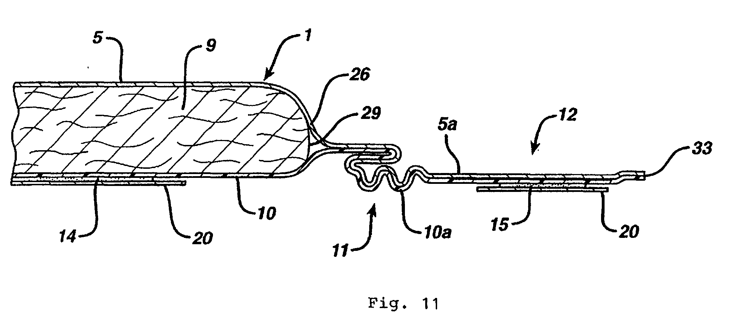

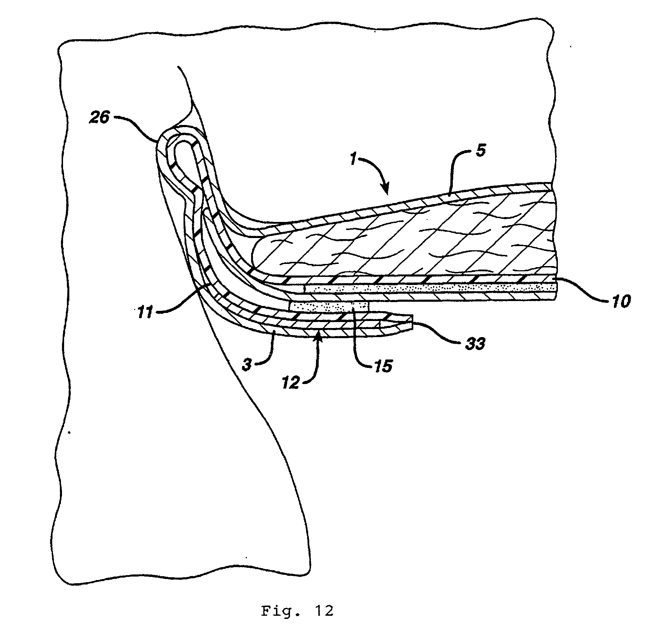

Referring to Figures 10-12, in an embodiment which is not according to the present invention, the flaps 3 having zones of differential stiffness are affixed to the barrier layer 10 of the sanitary napkin 1, inward of the longitudinal side edges 26 of the napkin. In this embodiment, the flexible portion 11 and the stiffened portion 12 enable the user to more easily gather or maintain the edges of the undergarment inward from the longitudinal side edges 26 of the napkin. Napkins having flaps 3 affixed to the barrier layer 10 of the napkin in accordance with this construction are more fully disclosed in co-pending patent application, U. S. Serial Number 08/772,343, (PCT/CA97/00990 filed December 22, 1997). The flaps 3 preferably have a flexible portion 11 having a width which is less than or equal to twice the distance in which the flaps 3 are affixed inward from the longitudinal side edges 26 of the napkin. That is, if the flaps 3 are affixed 4 millimeters inward from the longitudinal side edges 26, the flexible portion 11 should preferably have a width of about 8 millimeters or less. As shown in Figure 12, in this manner, the stiffened portion 12 of the flap will assure that the undergarment is maintained at a position which is inward from the longitudinal side edges 26 of the sanitary napkin 1.

The present invention is of general applicability in the field of disposable sanitary absorbent articles and generally includes, but is not limited to sanitary napkins, panty liners, incontinence devices, and the like.

## Claims

1. An absorbent article (1) for absorbing body exudate which is adapted to be worn in a crotch portion of a wearer's undergarment, the absorbent article comprising: an elongate main body portion (2) having opposite longitudinal side edges (26) and opposite transverse ends (27) which further comprises a body-facing fluid pervious topsheet (5), a garment-facing fluid impervious backsheet (10) and an absorbent core (9) between the topsheet (5) and the backsheet (10), and a pair of flaps (3) affixed to the longitudinal side edges (26) or to the backsheet (10), inward of the longitudinal side edges (26), along a line of juncture (29) in a central region of the main body portion (2) and extending laterally and terminating at a distal end (33), the flaps (3) being capable of being wrapped around the crotch portion of the wearer's undergarment, the flaps (3) having a flexible portion (11) and a stiffened portion (12), the flexible region being adjacent to and extending along the entire line of juncture (29) of the flap (3) and the main body (2) in the proximal region of the flap (3) , the stiffened portion (12) being adjacent to and extending laterally outward from the flexible portion (11), the stiffened portion (12) having a higher resistance to laterally compressive forces relative to the flexible portion (11), **characterized in that** the stiffened portion (12) includes a plurality of individual stiffened portions which are separated by one or more laterally extending flexible axes (13) and creates a preferential bending moment in the flexible portion (11) of the flap (3) when the flap (3) is folded around the crotch portion of the wearer's undergarment and wherein the flexible portion (11) has a width which is sufficient to permit the flexible portion (11) to form a smooth curve around the edge of the wearer's undergarment, and wherein the flexible portion (11) of the first flap (3) is separated from the flexible portion (11) of the second flap (3) by a distance that is between about 7 centimeters and 8.5 centimeters.

2. The absorbent article according to claim 1 wherein the absorbent article is selected from the group consisting of sanitary napkins, panty liners and incontinence devices.

3. The absorbent article according to claim 1 wherein the stiffened portion (12) extends continuously from the flexible portion (11) of the flap (3) to a freely extending distal end (33) of the flap (3).

4. The absorbent article according to claim 1 wherein the line of juncture (29) is nonlinear.

5. The absorbent article according to claim 1 wherein the flaps (3) are provided with zones of differential stiffness which are created by embossing the distal end regions of said flaps (3) in a pattern wherein the embossment (40) is a series of loops (41) which start adjacent to the flexible portion (11) and which extend outward the distal edge of the flaps (3) and curve backward toward the flexible portion (11) where they terminate.

6. The absorbent article according to claim 1 wherein the flexible portion (11) has a generally arcuate shape that allows it to conform to the increased front and back width of the panty crotch.

7. The absorbent article according to claim 1 wherein the stiffened portion (12) is formed by the incorporation into the flap (3) of a material selected from the group consisting of tissue, non-woven fabric, polymer film, airlaid pulp, polymeric foam, non-pressure sensitive adhesive, embossed patterns and combinations thereof.

8. The absorbent article according to claim 1 wherein the stiffened portion (12) is formed by a combination of a non-pressure sensitive adhesive and an embossed pattern.

9. The absorbent article according to claim 8 wherein the embossed pattern comprises a plurality of lateral lines (13) which extend from about the distal end of the flap (3) to about the line of juncture (29).

## Patentansprüche

1. Absorbierender Artikel (1), zur Absorption von Körperexsudaten, der geeignet ist, um in einem Schrittabschnitt von Unterwäsche eines Trägers getragen zu werden, wobei der absorbierende Artikel umfasst:
einen länglichen Hautkörperabschnitt (2), der gegenüberliegende Längsseitenränder (26) und gegenüberliegende Querenden (27) aufweist, der weiterhin eine, dem Körper zugewandte, flüssigkeitsdurchlässige Oberschicht (5), eine, der Kleidung zugewandte, flüssigkeitsundurchlässige Rückschicht (10) und einen absorbierenden Kern (9) zwischen der Oberschicht (5) und der Rückschicht (10), umfaßt.
und ein Paar von Laschen (3), die an den Längsseitenrändern (26) oder der Rückschicht (10), innerhalb von den Längsseitenrändern (26), entlang einer Verbindungslinie (29) in einem zentralen Bereich des Hauptkörperabschnitts (2) befestigt sind, und die sich lateral erstrecken und an dem distalen Ende (33) enden,
wobei die Laschen (3), um den Schrittabschnitt der Unterwäsche des Benutzers wikkelbar sind,
die Laschen (3), einen flexiblen Abschnitt (11) und einen versteiften Abschnitt(12) aufweisen,
wobei sich der flexible Bereich, entlang der gesamten Verbindungslinie (29) der Lasche (3) und des Hauptkörpers (2) im Nahbereich der Lasche (3) erstreckt und benachbart zu diesen befindet,
sich der versteifte Abschnitt (12) lateral nach außen von dem flexiblen Bereich (11) erstreckt und sich benachbart zu diesem befindet, sowie
der versteifte Abschnitt (12), einen höheren Widerstand gegen lateral komprimierende Kräße relativ zu dem flexiblen Abschnitt (11) aufweist,
**dadurch gekennzeichnet, daß**
der versteifte Abschnitt (12) eine Vielzahl von individuellen versteiften Bereichen enthält, die durch eine oder mehrere sich lateral erstreckende flexible Achsen (13) getrennt sind und ein bevorzugtes Biegemoment in dem flexiblen Abschnitt (11) der Lasche (3) erzeugen, wenn die Lasche (3) um den Schrittabschnitt der Unterwäsche des Benutzers gefaltet wird, und wobei der flexible Abschnitt (11) eine Breite aufweist, die ausreichend ist, um es dem flexiblen Abschnitt zu ermöglichen (11), eine glatte Kurve um den Rand der Unterwäsche des Benutzers zu formen, und wobei der flexible Abschnitt (11) der ersten Lasche (3) von dem flexiblen Abschnitt (11) der zweiten Lasche (3) durch einen Abstand getrennt ist, der ungefähr zwischen 7 cm und 8,5 cm beträgt.

2. Absorbierender Artikel nach Anspruch 1, **dadurch gekennzeichnet, daß** der absorbierende Artikel aus der Gruppe bestehend aus Damenbinden, Slipeinlagen und Inkontinenzvorrichtungen ausgewählt ist.

3. Absorbierender Artikel nach Anspruch 1, **dadurch gekennzeichnet, daß** sich der versteifte Abschnitt (12) durchgängig von dem flexiblen Abschnitt (11) der Lasche (3) zu einem sich frei erstreckenden distalen Ende (33) der Lasche (3) erstreckt.

4. Absorbierender Artikel nach Anspruch 1, **dadurch gekennzeichnet, daß** die Verbindungslinie (29) nicht linear ist.

5. Absorbierender Artikel nach Anspruch 1, **dadurch gekennzeichnet, daß** die Laschen (3) mit Zonen unterschiedlicher Steifheit versehen sind, die durch Einprägen des distalen Endbereichen der Laschen (3) in einem Muster erzeugt werden, wobei die Einprägung (40) eine Folge von Schlaufen (41) ist, die benachbart zu dem flexiblen Abschnitt (11) beginnen und sich nach außen von dem lateralen Rand der Lasche (3) erstrecken und sich zurück zu dem flexiblen Abschnitt (11) krümmen, wo sie enden.

6. Absorbierender Artikel nach Anspruch 1, **dadurch gekennzeichnet, daß** der flexible Abschnitt (11) eine allgemein bogenförmige Form aufweist, die es erlaubt, sich der größeren Vorder- und Rückbreite des Unterhosenschritts anzupassen.

7. Absorbierender Artikel nach Anspruch 1, **dadurch gekennzeichnet, daß** der versteifte Abschnitt (12) durch die Aufnahme eines Materials in die Lasche (3) geformt ist, das aus der Gruppe bestehend aus Gewebe, nicht gewebten Fasern, Polymerfilm, im Luftstrom aufgebrachter Zellstoff, Polymerschaum, nicht drucksensitivem Klebestoffe, eingeprägten Mustern und Kombinationen davon ausgewählt ist.

8. Absorbierender Artikel nach Anspruch 1, **dadurch gekennzeichnet, daß** der versteifte Abschnitt (12) aus einer Kombination von nicht drucksensitivem Klebstoff und einem eingeprägten Muster geformt ist.

9. Absorbierender Artikel nach Anspruch 8, **dadurch gekennzeichnet, daß** das eingeprägte Muster eine Vielzahl von lateralen Linien (13) umfasst, die sich ungefähr von dem distalen Ende der Lasche (3) zu ungefähr der Verbindungslinie (29) erstrecken.

## Revendications

1. Article absorbant (1) pour absorber l'exudat du corps qui est adapté pour être porté dans une partie du gousset d'un sous-vêtement de l'utilisatrice, l'article absorbant comprenant :une partie de corps principal allongée (2) dotée de bords latéraux longitudinaux opposés (26) et d'extrémités transversales opposées (27), qui comprend en outre une feuille de dessus perméable au fluide, orientée vers le corps (5), une feuille de dessous imperméable au fluide, orientée vers le vêtement (10) et un coeur absorbant (9) situé entre la feuille de dessus (5) et la feuille de dessous (10), et une paire de rabats (3) fixés aux bords latéraux longitudinaux (26) ou à la feuille de dessous (10), vers l'intérieur des bords latéraux longitudinaux (26), le long d'une ligne de jonction (29) située dans une région centrale de la partie de corps principal (2) et qui s'étendent de manière latérale et se terminent au niveau d'une extrémité distale (33), les rabats (3) pouvant être déformés autour de la partie de gousset du sous-vêtement de l'utilisatrice, les rabats (3) étant dotés d'une partie souple (11) et d'une partie rigide (12), la région souple étant adjacente à et s'étendant le long de toute la ligne de jonction (29) du rabat (3) et du corps principal (2) dans la région proximale du rabat (3), la partie rigide (12) étant adjacente à et s'étendant latéralement vers l'extérieur à partir de la partie souple (11), la partie rigide (12) présentant une résistance supérieure aux forces de compression de manière latérale par rapport à la partie souple (11), **caractérisé en ce que** la partie rigide (12) comprend une pluralité de parties rigides individuelles qui sont séparées par un ou plusieurs axes souples qui s'étend de manière latérale (13) et crée un moment de pliage préférentiel dans la partie souple (11) du rabat (3) lorsque le rabat (3) est plié autour de la partie de gousset du sous-vêtement de l'utilisatrice, et dans lequel la partie souple (11) présente une largeur qui est suffisante pour permettre à la partie souple (11) de former une courbe régulière autour du bord du sous-vêtement de l'utilisatrice, et dans lequel la partie souple (11) du premier rabat (3) est séparée de la partie souple (11) du second rabat (3) par une distance qui est comprise entre environ 7 centimètres et 8,5 centimètres.

2. Article absorbant selon la revendication 1 dans lequel l'article absorbant est sélectionné à partir du groupe qui se compose des serviettes hygiéniques, des protèges slip et des dispositifs contre l'incontinence.

3. Article absorbant selon la revendication 1, dans lequel la partie rigide (12) s'étend de manière continue à partir de la partie flexible (11) du rabat (3) vers une extrémité distale qui s'étend librement (33) du rabat (3).

4. Article absorbant selon la revendication 1, dans lequel la ligne de jonction (29) n'est pas linéaire.

5. Article absorbant selon la revendication 1, dans lequel les rabats (3) sont prévus avec des zones de rigidité différentielle qui sont créées en bosselant les régions de l'extrémité distale desdits rabats (3) selon un modèle dans lequel le bosselage (40) est une série de boucles (41) qui démarre de manière adjacente par rapport à la partie souple (11) et qui s'étend vers l'extérieur du bord distal des rabats (3) et se courbe vers l'arriére vers la partie souple (11) lorsqu'elle se termine.

6. Article absorbant selon la revendication 1, dans lequel la partie souple (11) présente une forme généralement arquée qui lui permet de s'adapter à la largeur plus importante avant et arrière du gousset du slip.

7. Article absorbant selon la revendication 1, dans lequel la partie rigide (12) est formée en incorporant dans le rabat (3) un matériau sélectionné à partir du groupe qui se compose de tissu, du tissu non tissé, du film polymère, de la pulpe airlaid, de la mousse polymère, de la colle sensible à la pression atmosphérique, des motifs en relief et des combinaisons de ceux-ci.

8. Article absorbant selon la revendication 1, dans lequel la partie rigide (12) est formée par une combinaison d'une colle sensible à la pression atmosphérique et d'un motif en relief.

9. Article absorbant selon la revendication 8, dans lequel le motif en relief comprend une pluralité de lignes latérales (13) qui s'étendent à partir d'environ l'extrémité distale du rabat (3) jusqu'à environ la ligne de jonction.
